# EUROPEAN PATENT APPLICATION

(11) **EP 1 731 597 A1**
(43) Date of publication of application: **13.12.2006**
(21) Application number: 05012162.3
(22) Date of filing: 06.06.2005
(51) Int. Cl.: C12N 5/00, A01K 61/00, C07K 14/435

(54) **Selenium-enriched liquid media for the cultivation of siliceous sponges and for biogenic silica production**

(71) Applicant: Johannes Gutenberg-Universität Mainz, 55122 Mainz (DE); BIOTECmarin GmbH, 55099 Mainz (DE)
(72) Inventor: Schwertner, Heiko, Dr., 19055 Schwerin (DE); Müller, Werner E.G., Professor Dr., 65203 Wiesbaden (DE); Schröder, Heinz, Prof. Dr.Dr., 65189 Wiesbaden (DE); Osinga, Ronald, Dr., 6718 HT Ede (NL)
(74) Representative: Krauss, Jan

(57) **Abstract**

The present invention generally relates to a defined medium composition for culturing sponges. The medium promotes growth in sponges and stimulates biogenic silica production in siliceous sponges. The present invention further relates to aspects in connection with said production.

## Description

### 1. Field of the invention

The present invention generally relates to a defined medium composition for culturing sponges. The medium promotes growth in sponges and stimulates biogenic silica production in siliceous sponges. The present invention further relates to aspects in connection with said production.

Since many years there is commercial interest in sponges, in particular marine sponges, due to ability of many of these animals to produce natural compounds that may be used as pharmaceutical drugs, cosmetic products or biomaterials. Many sponges are rare and therefore difficult to collect on a large scale. In addition, sponges are difficult to culture efficiently on a large scale.

The only existing method for mass cultivation of sponges is sea-based aquaculture (growing the animals in their natural environment, see for instance Duckworth and Battershill 2003, Dumdei et al. 1998, Hadas et al. 2005, Müller et al. 1999a, Pronzato et al. 1999, Schatton et al. ― German patent DE 198 34 949 A1). Due to unpredictable and uncontrollable circumstances that prevail outdoors, sea-based aquaculture is a risky technology: outdoors, the cultured animals are subjected to disasters such as storms, oil spills and diseases and often, the conditions outdoors are suboptimal (for instance in winter, when both the availability of food and the temperature are low). Besides, it is often hard to find a location that is suitable for aquaculture.

For aquatic invertebrates other than sponges, land-based aquaculture in controllable closed and half-open systems is a commonly used alternative to sea-based culture. A wide variety of land-based systems has been applied to culture fish, shrimp and several of other marine animals. Currently, neither a land-based aquaculture system nor an *in vitro* culture system exists that enables mass production of sponges and their metabolites. The present invention generally relates to liquid culture media that enable mass cultivation of sponges in land-based systems (being either aquaculture systems or *in vitro* culture systems).

### 2. Background of the invention

In recent years, it became evident that the biochemical composition of liquid media for land-based sponge culture is strongly influencing important physiological and morphogenetic processes. Studies with sponge primmorphs *(in vitro* cultured sponge cell aggregates that contain proliferating cells ― see Müller et al. 1999b) showed that the trace element composition of the medium affects sponge physiology up to a molecular level. Silicon, for instance, promoted the formation skeletal components in sponge primmorphs by inducing genes that code for the enzymes myotrophin and silicatein (Krasko et al. 2000). Myotrophin induces protein synthesis, in particular collagen, which is a major component of sponges. Silicatein is involved in the formation of siliceous spicules, these are skeletal components that consist of biogenic silica. Enrichment of the culture media with ferric iron further stimulated spicule formation (Krasko et al. 2002, LePennec et al. 2003). The expression level of silicatein is significantly higher in primmorphs that are cultured in ferric iron enriched media. Ferric iron also induced channel formation in primmorphs, which is a morphological characteristic of sponges and is promoting cell proliferation during the early formation of a primmorph from dissociated sponge cells. In addition to these observations, it was found that ferric iron promotes the regeneration of an aquiferous system in fragments of functional sponges (R.Osinga, unpublished results).

Application of the information above has up till now not resulted in reproducible methods for land-based aquaculture and *in vitro* culture of sponges. It appears that hitherto unknown factors have been overlooked.

### 3. Summary of the invention

The present invention in one aspect thereof relates to culture media based on freshwater, brackish water, natural seawater or artificial seawater (salinity ranging from 0 to 50 ‰). The water is enriched with the elements silicon (final concentration in the medium between 1 and 100 µM Si), ferric iron (final concentration in the medium between 1 and 100 µM Fe3+) and selenium (final concentration in the medium between 1 and 1000 µM Se). Silicon can be added to the medium as either silicon salts (e.g. Sodium metasilicate), silicon containing natural (silicon sand) or artificial materials (glass), or silicon-enriched commercial media. Ferric iron can be added to the medium as either ferric iron salts (e.g. ferric citrate), ferric iron containing natural or artificial materials, or ferric iron-enriched commercial media. Selenium can be added to the medium as either selenium salts (e.g. sodium selenite), selenium containing natural or artificial materials, or selenium-enriched commercial media (e.g. selenite-broth).

The media can contain an organic food source, which can be anything that has been, or will be demonstrated to be appropriate for sponges (for example: the marine microalga *Phaeodactylum tricornotum,* commercial cell culture media such as DMEM and RPI).

The media can be used to culture sponges, in particular siliceous sponges and can be applied to promote biosilica formation in siliceous sponges or *in vitro* by (recombinant) sponge enzymes.

Thus, the present invention in a first aspect thereof relates to a culture medium for the land-based aquaculture *or in vitro* culture of sponges, comprising between 10 and 1000 µM Se. Preferably, said culture medium according to the invention further comprises between 10 and 100 µM Si, and between 10 and 100 µM Fe3+.

More preferably, said culture medium according to the invention is based on freshwater, brackish water, natural seawater or artificial seawater with a salinity ranging from 0 to 50 ‰. More preferably, to said culture medium according to the invention silicon is added to the medium as silicon salts such as sodium metasilicate, silicon containing natural materials such as silicon sand or artificial materials such as glass, or silicon-enriched commercial media. Still more preferably, to said culture medium according to the invention the ferric iron is added to the medium as either ferric iron salts such as ferric citrate, ferric iron containing natural or artificial materials, or ferric iron-enriched commercial media. Still more preferably, to said culture medium according to the invention wherein the selenium is added to the medium as either selenium salts such as sodium selenite, selenium containing natural or artificial materials, or selenium-enriched commercial media such as selenite-broth. Still more preferably, said culture medium according to the invention further contains an organic food source, such as, for example the marine microalga *Phaeodactylum tricornotum,* or commercial cell culture media such as DMEM or RPI.

The present invention in a next aspect thereof relates to a method for biogenic silica formation comprising cultivating sponges, sponge fragments, sponge primmorphs, sponge cells in a culture medium according to the invention, and/or comprising an enzymatic biosilica formation comprising sponge-derived enzymes comprising incubation in a culture medium according to the invention. Preferably, said method according to the invention, comprises an induction of the silicatein associated protein according to SEQ ID No. 1.

The present invention in a next aspect thereof relates to a silicatein associated protein comprising the amino acid sequence according to SEQ ID No. 1, and biologically functional variants thereof. Biologically functional variant means truncated or mutated enzymes based on SEQ ID No. 1 that still are able to perform the enzymatic function(s) of the amino acid sequence according to SEQ ID No. 1. Variants also include identical enzymes from other species, such as, for example, other marine sponges. The present invention in a next aspect thereof relates to a nucleic acid molecule encoding for the silicatein associated protein according to the invention, in particular according to the nucleic acid sequence according to SEQ ID No. 2.

The present invention in a next aspect thereof relates to a silicatein associated protein comprising the amino acid sequence according to SEQ ID No. 3, and biologically functional variants thereof. Biologically functional variant means truncated or mutated enzymes based on SEQ ID No. 3 that still are able to perform the enzymatic function(s) of the amino acid sequence according to SEQ ID No. 3. Variants also include identical enzymes from other species, such as, for example, other marine sponges. The present invention in a next aspect thereof relates to a nucleic acid molecule encoding for the silicatein associated protein according to the invention, in particular according to the nucleic acid sequence according to SEQ ID No. 4.

Both silicatein associated proteins according to the present invention can be used for an effective biogenic silica formation, in particular in an in vitro system and/or cell culture.

The present invention in a next aspect thereof relates to a method for producing a culture medium for the land-based aquaculture or *in vitro* culture of sponges, comprising adding of between 10 and 1000 µM Se.

The present invention in a next aspect thereof relates to the use of a culture medium according to the invention for biogenic silica formation comprising cultivating sponges, sponge fragments, sponge primmorphs, sponge cells in, and/or comprising an enzymatic biosilica formation comprising sponge-derived enzymes. Preferred is a use according to the invention, wherein selenium is present in said medium at between 10 and 1000 µM Se.

### 4. Detailed description of the invention

The necessity to enrich a sponge culture medium with silicon and ferric iron is apparent from the finding described in literature (reviewed by LePennec et al. 2003). The combination of these enrichments with selenium-enrichment is novel. The rationale for using selenium is explained below.

It was demonstrated (see section 4.2 for details) that selenium-dependent t-RNAs exist in sponges. Selenium-dependent t-RNA translates a cysteine codon as seleno-cysteine (in this cysteine derivative, the sulphur atom is replaced by a selenium atom). The t-RNA molecule itself also encompasses a selenocysteine moiety. During the translation process, the t-RNA can overwrite a TGA stop-codon and incorporates its selenocysteine moiety into the protein that is being synthesised (Low and Berry 1996). Due to these features, selenium is needed for the biosynthesis of a number of specific proteins. As such, the presence of selenium is considered essential for the physiology of sponges.

Selenium is not only needed as a building block for selenocysteine (which is incorporated in a number of proteins and in the selenium dependent t-RNA molecule itself), it is also involved in the translation of genes encoding selenium containing proteins. This was demonstrated by gene expression studies in sponge primmorphs after exposure to elevated selenium concentrations (see section 4.2 for details).

Two sponge proteins containing selenocystein have been characterised (see section 4.1 for details). Both are involved in the formation of spicules. Hence, selenium is essential for the formation of skeletal elements in siliceous sponges that produce spicules. Since the growth of these sponges cannot continue without the formation of these skeletal components, selenium can be considered as an essential element for the growth, and hence, the cultivation of these sponges.

The present invention will now be described further in the following examples with reference to the accompanying figures and sequences without being limited thereto. In the figures,

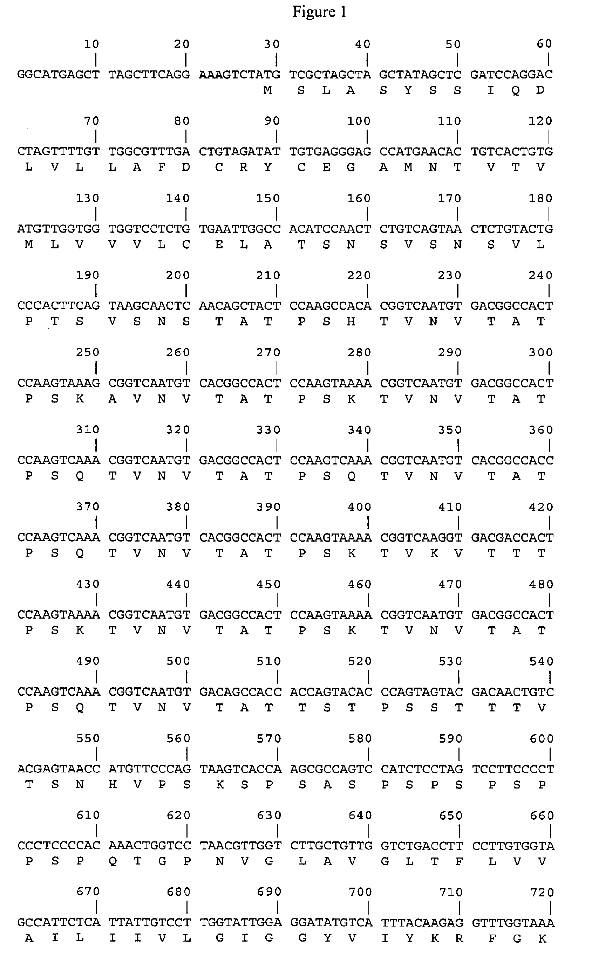

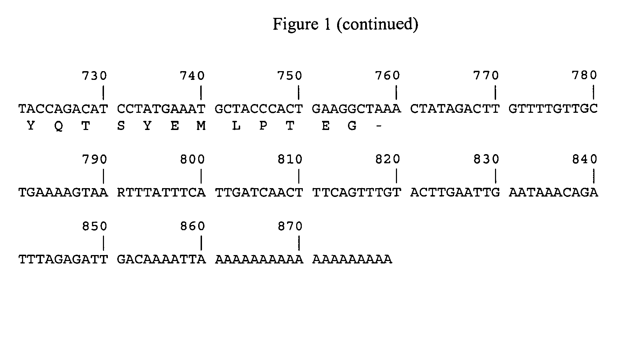

Figure 1 shows the nucleotide and amino acid sequences of the silicatein-associated protein SILaP_SUBDO according to the invention. The sequence are further identified in SEQ ID No. 1 (amino acid sequence) and SEQ ID No. 2 (nucleotide sequence), respectively.

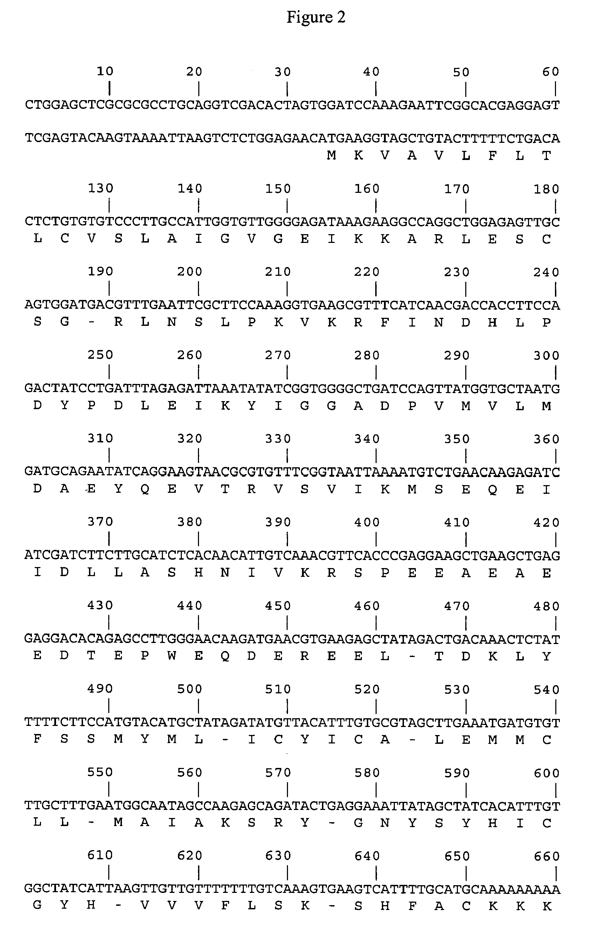

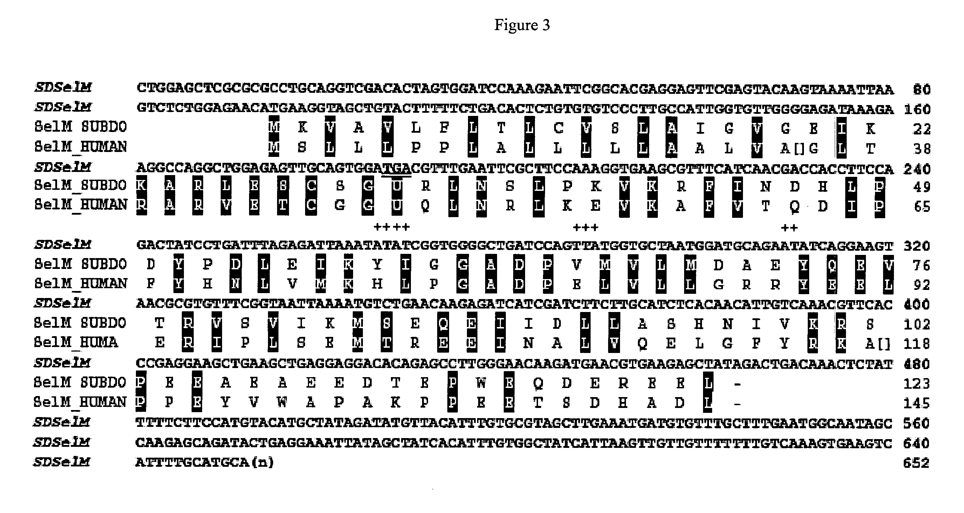

Figure 2 shows the nucleotide and amino acid sequences of the *Suberites domuncula* selenoprotein M according to the invention. The sequence are further identified in SEQ ID No. 3 (amino acid sequence) and SEQ ID No. 4 (nucleotide sequence), respectively. Note that the precursor site at position 32 has been omitted from the sequence (see Figure 3 for details).

Figure 3 shows *Suberites domuncula* selenoprotein M. From the S. *domuncula* nt sequence (*SDSelM*) the selenoprotein M protein (SelM_SUBDO) is predicted and aligned with the human selenoprotein M precursor (SelM_HUMAN, NP_536355); the human sequence was shortened between aa₂₀ to aa₃₅ and aa₁₁₉ to aa₁₂₄ []). Residues conserved (similar or related with respect to their physico-chemical properties) in both sequences are shown in white on black; the characteristic nucleotide invariances around the SECIS element with the TGA triplet in the center are marked (+). The TGA triplet that encodes selenocysteine (U) is underlined.

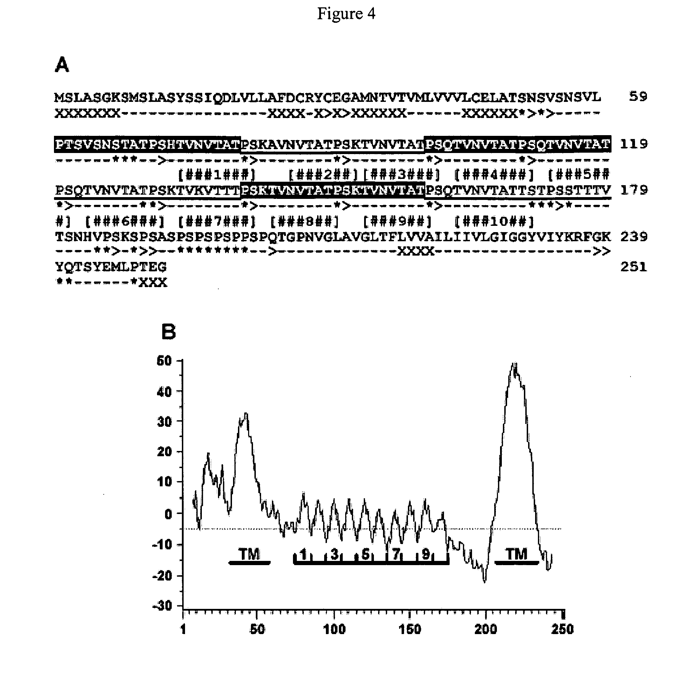

Figure 4 shows Silicatein-associated protein SILaP_SUBDO. (A) The deduced protein was analyzed for the predicted secondary structure according to the method described by Garnier et al. (Garnier, J., Osguthorpe, D.J. & Robson, B. (1978) Predicting the secondary structure of globular proteins. J. Mol. Biol. 120, 97-120); the helical conformation (X), the extended conformation (-), the turn (>) as well as the coil conformation (*) are indicated. The six highly similar segments of 20 amino acids are marked in white on black or are underlined. In addition, the 10 hydrophobic regions, present in the six 20 amino acids long blocks, are indicated and numbered (#). (**B**) Hydropathicity plot of the *S. domuncula* SILaP_SUBDO; the calculation was performed according to the method of Kyte and Doolittle (Kyte, J. & Doolittle, R.F. (1982) A simple method for displaying the hydrophobic character of a protein. J. Mol. Biol. 157, 105-132). The horizontal axes show the aa numbers along the protein versus the corresponding hydropathicity. The dotted lines at the -5 value divide hydrophobic regions (above) from hydrophilic regions (below). The 10 distinct hydrophobic regions are consecutively labeled. The two highly hydrophobic (potential transmembrane; TM) regions were identified; they range from aa₃₂ to aa₅₅ and from aa₂₀₄ to aa₂₃₇.

### Examples

### 4.1. Demonstration of the presence of genes encoding selenium dependent proteins in Suberites domuncula

### Materials & Methods

Primmorphs of the Mediterranean sponge *Suberites domuncula* were prepared according to Muller et al. (1999). Seven days old primmorphs were incubated in natural seawater supplemented with 0.25% RPMI 1640 / 0.75% DMEM medium (hereafter referred to as Standard Medium). Series of primmorphs were incubated in Standard Medium supplemented with:
- 30 µM silicate and 10 µM iron (III)
- 30 µM silicate, 10 µM iron (III) and 10 to 1000 µM sodium selenite
- 10 to 1000 µM sodium selenite

Extracts from primmorphs were prepared by homogenization in lysis-buffer (1x TBS [Tris-buffered saline], pH 7.5, 1 mM EDTA, 1% Nonidet-P40, protease inhibitor cocktail [1 tablet/10 ml]). After centrifugation, the supernatants were subjected to subsequent analysis.

*Differential display technique.* The technique of differential display was applied to identify the changes in the expression levels of transcripts as described (Müller et al., 2002; Müller et al., 2003a). Total RNA (1 µg) from controls as well as from selenium treated samples was reverse-transcribed using an equimolar oligonucleotide mixture of dT₂₃N (Sigma; N represents G, C or A) as 3'-primer in a reaction with the Moloney murine leukemia virus reverse transcriptase (Promega, Madison, WI; USA) at a reaction temperature of 37°C. The resulting cDNA was used as template for the subsequent polymerase chain reaction (PCR) with the 5'-IRD 800 infrared labeled arbitrary primer 5'-AGTGAATGCG-3' and one of the dT₂₃N primers in the assay. To avoid any DNA based contamination through the reagents, the assay was incubated [prior to the addition of the template] with the restriction enzyme *Sau*3AI for 20 min at 37°C followed by a final inactivation step at 72°C for 10 min. The PCR parameters were used: 94°C for 3 min, 45 cycles of 94°C for 30 sec - 40°C for 2 min - and 72°C for 30 sec, with an additional extension step at 72°C for 10 min. After amplification the labeled fragments were separated on a 6% polyacrylamide sequencing gel using a DNA sequenator (Li-Cor 4000S). The sequencing run was stopped after 3 hrs and the gel was transferred on millimeter paper and vacuum dried. The differences in the banding pattern on the gel were detected by using an infrared scanning device (Odyssey, LiCor; Lincoln, NE; USA). The major DNA bands, which were differentially seen in the assays from selenite were excised and diluted in distilled water. The resulting fragments were subsequently re-amplified, subcloned in *pCRII*-Topo vector (Invitrogen, Carsbad, CA; USA) and sequenced using the "Thermo-Sequenase Fluorescent Labeled Primer Cycle Sequencing Kit" (Amersham/Pharmacia, Little Chalfont, Buckinghamshire; UK) and the sequencing device (LongReadIR 4200, Li-Cor).

The sequences of the cDNAs obtained from differential display were analyzed using computer programs BLAST (2003) and FASTA (2003).

### Results

Exposure to elevated selenium levels resulted in the upregulation of over 30 DNA sequences in primmorphs of S. *domuncula.* Among the sequences obtained, three partial cDNAs were isolated whose deduced polypeptides showed sequence similarity to the human selenoprotein M.

The complete cDNA was obtained by primer walking (Ausubel et al., 1995; Wiens et al., 1998). The complete protein, with a calculated Mᵣ of 13,918 shares the highest sequence similarity with the 15 kDa selenoprotein M from human (Korotkov et al. 2002; accession number NP_536355M). Therefore, the sponge molecule was termed selenoprotein M (SelM_SUBDO), and its cDNA *SDSelM.* The 652 nts long cDNA (accession number AJ875186) contained one ORF, spanning nt₉₄₋₉₆ to nt₄₆₃₋₄₆₅₍ₛₜₒₚ₎. One TGA stop codon exists at nt₁₈₇₋₁₈₉ which can function also as a codon for selenocysteine (Low and Berry 1996). This triplet is surrounded by the conserved selenocysteine insertion sequence (SECIS) element (Low and Berry 1996). According to the critical features, the one in the sequence here belongs to the form 2 SECISs (Martin 2001). This suggests that the TGA stop codon may be suppressed and (very likely) be used for the insertion of selenocysteine.

A further differentially expressed transcript was characterized, which encoded a sponge-specific protein that was termed silicatein-associated protein (SILaP_SUBDO). The corresponding cDNA, *SDSILαP,* had a length of 860 bp and comprises one ORF, which is found between nt₄₋₆ to nt₇₅₇₋₇₅₉ (stop) (accession number AJ872182). The 251 aa long polypeptide has a calculated Mᵣ of 25,602. This protein displayed no striking homology to any protein reported in the database.

### Conclusions

Selenium has a profound effect on gene expression patterns in *Suberites domuncula,* since over 30 sequences are upregulated after exposure of sponge materials to this element. At least one of the upregulated genes is encoding a sponge-specific protein that contains selenocysteine. This gene comprises features that suggest the existence of Se-dependent t-RNA in the sponge. Hence, selenium is likely to play an essential role in the biochemistry and molecular biology of the sponge.

### 4.2. Transcription and translation of selenium-dependent genes: demonstration of the presence of selenium-dependent t-RNAs and the location of selenium containing proteins in Suberites domuncula

### Materials & Methods

*Preparation of recombinant selenoprotein M and silicatein-associated protein.* Expression of selenoprotein M: The sponge SDSelM sequence was expressed in *Escherichia coli* (TOP10). The open reading frame (ORF; nt₂₁₀ to nt₄₆₂; corresponding to aa₄₀ to aa₁₂₃ of the mature protein), was isolated by PCR using one forward primer (5'-ccatggcaaccaaaccctctggtccta-3' [the NcoI restriction site is underlined]) and one reverse primer (5'-aagcttagatacttcttgactttcacc-3') [HindIII]). The 252 bp long part was cloned into the expression vector pBAD/gIII A (Invitrogen), which contained at the 3'-terminus the myc epitope and the polyhistidine region. The insert was expressed overnight at 30°C in the presence of 0.0002% of L-arabinose. The fusion protein was extracted and purified with the His•tag purification kit (Novagen, Madison WI; USA). The purity of the material was checked by 10% polyacrylamide gels containing 0.1 % NaDodSO₄ (PAGE) according to Laemmli (1970). The recombinant selenoprotein M, r-SelM, was dialyzed against 25 mM Tris-HCl buffer (pH 7.2), supplemented with 10 mM of DL-dithiothreitol.

Expression of Silicatein-associated protein: For the preparation of the recombinant protein of silicatein-associated protein, r-SILaP, the same vector with the complete ORF of the SDSILaP cDNA was used; again the cDNA was inserted into the vector at same restriction sites, the NcoI and HindIII. Expression and purification procedures were the same as for selenoprotein M.

*Raising of antibodies.* Polyclonal antibodies (PoAb) were raised against the recombinant r-SelM and r-SILaP in female rabbits (White New Zealand) as described (Schütze et al. 2001); the PoAb were termed PoAb-SelM and PoAb-SILaP. In control experiments 100 µl of the PoAb-SelM or PoAb-SILaP were adsorbed to 20 µg of r-SelM or r-SILaP (30 min; 4°C) prior to use. The preparation of antibodies against recombinant silicatein was described before (Müller et al. 2003b); it was termed PoAb-SILIC. It was raised against the long form of the r-silicatein (Mᵣ 35 kDa).

*Western blotting.* Protein samples, extracts from primmorphs and axial filaments were analyzed. Samples of 5 µg protein (axial filament) or 20 µg extract were dissolved in loading buffer (Roti-Load; Roth, Karlsruhe; Germany), boiled for 5 min, subjected to NaDodSO₄₋PAGE (10% polyacrylamide and 0.1% NaDodSO₄) and electrophoresed as described (Laemmli, 1970). The gels were stained with Coomassie brilliant blue. In parallel, the proteins were transferred, the PVDF membranes (Millipore-Roth) were incubated with PoAb-SILIC, PoAb-SelM or PoAb-SILaP (1:500 dilution); the immune complexes were visualized by incubation with anti-rabbit IgG (alkaline phosphatase conjugated), followed by staining with 4-chloro-1-naphthol. As size markers Multi-Tag-Markers (Roche Mannheim, Mannheim; Germany) were used.

*RNA preparation and Northern blot analysis.* RNA was extracted from liquid-nitrogen pulverized tissue with TRIzol Reagent (GibcoBRL, Grand Island, N.Y.) as described (Grebenjuk et al., 2002). Then 5 µg of total RNA was electrophoresed and blotted onto Hybond-N⁺ nylon membrane (Amersham). Hybridization was performed with a 250 nt large part of the SDSelM cDNA, a 300 nt segment of SDSILaP and a 400 nt fragment from silicatein-α gene SDSILIC (Krasko et al. 2000; accession number AJ272013). The probes were labeled with the PCR-DIG-Probe-Synthesis Kit (Roche). After washing DIG-labeled nucleic acid was detected with anti-DIG Fab fragments and visualized by chemiluminescence technique using CDP (Roche).

*Immunohistochemistry.* Fresh sponge tissue from the Adriatic Sea was fixed in 2% paraformaldehyde (Romeis 1989); after dehydration the samples were embedded in Technovit 8100 (Beckstead 1985), according to the instructions of the manufacturer as outlined in Pancer et al. (1996). Sections of 8 µm thickness were prepared. The sponge tissue was ― on purpose ― not treated with NaHF/FH solution in order not to remove the siliceous spicules. The sections were blocked with 1% bovine serum albumin (BSA) in phosphate-buffered-saline (PBS) and incubated with the antibody against selenoprotein M (PoAb-SelM), silicatein-associated protein (PoAb-SILaP) or silicatein (PoAb-SILIC) overnight at 4 °C. The antibodies were used in a 1:200 dilution in 0.5% BSA in PBS buffer. Rhodamine-conjugated goat anti-rabbit immunoglobulin (Dako, Carpinteria; USA) was used as a secondary antibody. The pre-immune rabbit serum was used as a control; they did not react with any structures in the sections. For light microscopical studies (immunofluorescence analysis) an Olympus AHBT3 microscope was used. The fluorescence studies were performed with an AH3-RFC reflected light fluorescence attachment using the excitation light wave-length 334/365 nm. In parallel, the non-stained sections were inspected directly using Nomarsky interference contrast optics.

### Results

*Protein expression of selenoprotein M after exposure to selenium.* Extracts of primmorphs were prepared and subjected to Western blot analysis. The blotting results revealed that in the absence of selenium the 13 kDa-selenoprotein M is missing from the extract. Also an incubation of 24 hrs with Na-selenite did not resulted in a change of the expression pattern. However, after 48 hrs and especially 72 hrs the 13 kDa band, reflecting selenoprotein M, was markedly present on the immunoblot.

*Gene expression studies.* The effect of selenium on the expression of the genes encoding selenoprotein M, silicatein-associated protein and silicatein was studied. In the absence of Na-selenite almost no transcripts could be detected during the 72 hrs incubation period for *selenoprotein M* (the probe *SDSelM* was used) and *silicatein-associated protein* (*SDSILaP*), while a high level of *silicatein* (*SDSILIC*) transcripts could be detected by Northern blotting. However, during the 72 hrs-incubation period with Na-selenite, within the concentration range from 10 µM to 1000 µM, a strong upregulation of the expression of the genes for *selenoprotein M* and *silicatein-associated protein* was detectable. Based on the data obtained, the steady state expression was almost identical within the selenium concentration range chosen. The expression level of *silicatein* remained almost unchanged during the exposure to selenium.

*Identification of selenoprotein M, silicatein-associated protein and silicatein in total extract or in axial filaments by Western blotting.* In order to clarify if the protein selenoprotein M and silicatein-associated protein are associated with the axial filaments of the spicules from S. *domuncula* Western blot/antibodies studies were performed. Extracts from Na-selenite-treated primmorphs and axial filaments from spicules were size separated and subjected to Western blot experiments. The blots were incubated with antibodies against selenoprotein M (PoAb-SelM), silicatein-associated protein (PoAb-SILaP), or silicatein (PoAb-SILIC).

Using these tools it could be demonstrated that selenoprotein M exists in larger amounts in the soluble extracts and to a small extent also in the axial filaments. Likewise, also the silicatein-associated protein was identified in the total extracts and also in lower amounts in the axial filament. As recently demonstrated (Müller et al., submitted), and here used as an internal control, silicatein exists predominantly in the axial filaments.

*Localization of selenoprotein M and silicatein-associated protein by immunofluorescence analysis.* The localization of the proteins under investigation in tissue from S. *domuncula* was performed using the antibodies PoAb-SILIC, PoAb-SelM and PoAb-SILaP as tools. Sections were performed through tissue from which the spicules had not been removed. The sections were incubated with antibodies against selenoprotein M (PoAb-SeIM), silicatein-associated protein (PoAb-SILaP) or silicatein (PoAb-SILIC).

Fluorescence images, obtained with antibodies against silicatein showed that primarily/exclusively the surfaces of the 5 to 7 µm thick and up to 450 µm long monactinal tylo-styles and to a smaller extent the diactinal oxeas and also the axial filaments are recognized by PoAb-SILIC. This finding is interesting, since it demonstrates that silicatein is not restricted alone to the axial filament but that this enzyme exists also around the spicules.

Images obtained with the antibodies raised against selenoprotein M and silicatein-associated protein surprisingly revealed strong immunoreactions not only on the surfaces of the spicules but also in the canals of them which harbor the axial filaments. Parallel Nomarsky interference images showed that, in addition to these structures, also areas in the mesohyl of the tissue are decorated by the antibodies.

### Conclusions

Gene expression patterns in primmorphs exposed to selenium and immunochemistry analysis of functional sponge tissue revealed that the genes encoding selenium dependent proteins are transcribed and translated into functional proteins. Hence, the selenocysteine tRNA population exist in the sponge.

The detailed biological role of selenoprotein M in higher metazoan phyla is not known. Immunohistochemical analysis revealed that the sponge selenoprotein M is localized primarily in the axial filament and the surfaces of the spicules. This intriguing finding might suggest that selenoprotein M functions as an enzyme, which is likely to be involved in spiculogenesis.

In *S. domuncula* the increase of *selenoprotein M* expression at concentrations of 10 µM and higher is drastic. It is concluded that the steady level of selenoprotein M and of silicatein-associated protein in primmorphs is controlled by selenium on the transcriptional level. This finding is surprising, since in vertebrates, the expression of all selenoproteins is regulated on the level of translation (Sunde 2001). Hence, the role of selenium in sponge physiology differs from the physiological functions of selenium that are known up till now from other organisms.

### 4.3. Effects of selenium on enzymatic biosilica formation

### Materials & Methods

The same series of incubations of primmorphs as described in section 4.1 was used to determine the effect of selenium on biosilica formation in primmorphs (Standard Medium + iron (III) + silicate, Standard Medium + iron (III) + silicate + selenite, Standard Medium + selenite). In addition, effects of selenium on biosilica formation by free silicatein were studied using the recombinant silicatein [r-silicatein] described by Krasko et al. (2002). The reactions were performed on a glass slide in a volume of 100 µl. The reaction contained 10 µg of r-silicatein in phosphate-buffered-saline [PBS] (pH 7.2; including 5 mM Fe[+++] and 1 µM ZnCl₂). As substrate 4.5 mM tetraethoxysilane (TEOS; Sigma) was used. The reaction was performed at 22°C for 3 hrs and then washed 3-times with PBS. 1 µM of Na-selenite was added to the reaction. This was compared to a non-supplemented control. Rhodamine 123 (Sigma) was used to stain silica aggregates formed, as described (Li et al. 1989). Finally, the specimens were analyzed with an Olympus AHBT3 microscope/AH3-RFC light fluorescence using the excitation light wave-length 490 nm.

*Silica content in primmorphs.* To determine semi-quantitatively the silica content in primmorphs, these 3D-cell aggregates were disintegrated with Na-hypochlorite (10% for 24 hrs at room temperature). The particles, mainly spicules and precursors of them, were obtained by centrifugation, treated with NaOH and the amount of released silicic acid was determined colorimetrically with the molybdate assay (Cha et al. 1999), using the "Silicon Test" as described by the manufacturer (Merck, Darmstadt; Germany).

### Results

If 10 µM Na-selenite is added together with silicic acid and iron-III to the primmorphs a duplication of the silica concentration after 7 days is seen (32 ± 12 mg/g), while no stronger increase is measured after 14 days (34 ± 12 mg/g). If Na-selenite is added alone, in the absence of additional silicic acid and iron-III, no significant change in the amount of silica is measured in the primmorphs.
Selenium had a profound effect on biosilica formation *in vitro:* in the absence of the TEOS substrate only small aggregates, < 3 µm, could be visualized under fluorescence light. If TEOS was added to the assays with the recombinant silicatein the size of the aggregates increased to 10 µm after an incubation period of 15 min. Longer incubation for 3 hrs resulted in a further growth of the aggregates to 30 - 50 µm. If during this 3 hrs incubation period 1 µM of Na-selenite was present in the reaction the sizes of the aggregates reached values of 50-100 µm.

### Conclusions

Selenium not only stimulates spicule formation via incorporation into selenoproteins involved in spiculogenesis, but also has a direct stimulatory effect on silicatein, as was demonstrated by the two-fold increase in biosilica formation *in vitro.* The results clearly demonstrate that selenium is stimulating spicule formation only when applied in combination with silicic acid and iron-III.

### References

Ausubel, F.M., Brent, R., Kingston, R.E., Moore, D.D., Smith, J.A., Seidmann, J.G. & Struhl K. (1995) Current Protocols in Molecular Biology. John Wiley and Sons, New York
Beckstead, J. H. (1985) Optimal antigen localization in human tissues using aldehydefixed plastic-embedded sections. J. Histochem. Cytochem. 9, 954-958.
BLAST. 2003. http://www.ncbi.nlm.nih.gov/blast/blast.cgi: INTERNET.
Cha, J.N., Shimizu, K., Zhou, Y., Christianssen, S.C., Chmelka, B.F., Stucky, G.D. & Morse, D.E. (1999) Silicatein filaments and subunits from a marine sponge direct the polymerization of silica and silicones in vitro. Proc. Natl. Acad. Sci. USA 96, 361-365.
Dumdei, E.J., Blunt, J.W., Munro, M.H.G., Battershill, C.N. & Page, M.J. (1998) The whys and whats of sponge chemistry: why chemists extract sponges and what problems does this cause. In: Watanabe, Y. & Fusetani, N. (Eds), Sponge Sciences: Multidisciplinary Perspectives. Springer-Verlag, Tokyo, pp. 353-364.
Duckworth, A.R. & Battershill, C.N. (2003) Sponge aquaculture for the production of biologically active metabolites: the influence of farming protocols and environment. Aquaculture 221: 311-329.
FASTA. 2003. http://www.ncbi.nlm.nih.gov/BLAST/fasta.html: INTERNET.
Grebenjuk, V.A., Kuusksalu, A., Kelve, M., Schütze, J., Schröder, H.C. & Müller, W.E.G. (2002) Induction of (2'-5')oligoadenylate synthetase in the marine sponges Suberites domuncula and Geodia cydonium by the bacterial endotoxin lipopolysaccharide. Europ. J. Biochem. 269, 1382-1392.
Hadas, E., Shpigel, M. & Ilan, M. (2005) Sea ranching of the marine sponge Negombata magnifica (Demospongiae, latrunculidae) as a first step for latrunculin B mass production. Aquaculture 244: 159-169.
Korotkov, K.V., Novoselov, S.V., Hatfield, D.L. & Gladyshev, V.N. (2002) Mammalian selenoprotein in which selenocysteine (Sec) incorporation is supported by a new form of Sec insertion sequence element. Mol. Cell. Biol. 22, 1402-1411.
Krasko, A., Batel, R., Schröder, H.C., Müller, I.M. & Müller, W.E.G. (2000) Expression of silicatein and collagen genes in the marine sponge Suberites domuncula is controlled by silicate and myotrophin. Europ. J. Biochem. 267, 4878-4887.
Krasko, A., Schröder, H.C., Batel, R., Grebenjuk, V.A., Steffen, R., Müller, I.M. & Müller, W.E.G. (2002) Iron induces proliferation and morphogenesis in primmorphs from the marine sponge Suberites domuncula. DNA & Cell Biol. 21, 67-80.
Laemmli, U.K. (1970) Cleavage of structural proteins during the assembly of the head of bacteriophage T4. Nature 227:680-685.
LePennec, G., Perovic, S., Ammar, M.S.A., Grebenjuk, V.A., Steffen, R., Brümmer, F. & Müller, W.E.G. (2003) Cultivation of primmorphs from the marine sponge Suberites domuncula: morphogenetic potential of silicon and iron. Journal of Biotechnology 100: 93-108.
Low, S.C. Berry, & M.J. (1996) Knowing when not to stop: selenocysteine incorporation in eukaryotes. Trends Biochem. Sci. 21, 203-208.
Martin, G.W. (2001) SECIS elements. In Selenium - its Molecular Biology and Role in Human Health (Hatfield, D.L., ed.), pp 45-67. Kluwer Academic Publishers, Boston.
Müller, W.E.G., Wimmer, W., Schatton, W., Böhm, M., Batel, R. & Filic, Z. (1999a) Initiation of an aquaculture of sponges for the sustainable production of bioactive metabolites in open systems: example, Geodia cydonium. Mar. Biotechnol. 1: 569-579.
Müller, W.E.G., Wiens, M., Batel, R., Steffen, R., Borojevic, R. & Custodio, M.R. (1999) Establishment of a primary cell culture from a sponge: primmorphs from Suberites domuncula. Marine Ecol. Progr. Ser. 178, 205-219.
Müller, W.E.G., Krasko, A., Skorokhod, A., Bünz, C., Grebenjuk, V.A., Steffen, R., Batel, R., Müller, I.M. & Schröder, H.C. (2002) Histocompatibility reaction in the sponge Suberites domuncula on tissue and cellular level: central role of the allograft inflammatory factor 1. Immunogenetics 54, 48-58.
Müller, W.E.G., Klemt, M., Thakur, N.L., Schröder, H.C., Aiello, A., D'Esposito, M., Menna, M. & Fattorusso, E. (2003a) Molecular/chemical ecology in sponges: evidence for an adaptive antibacterial response in Suberites domuncula. Marine Biol. 144, 19-29.
Müller, W.E.G., Krasko, A., Le Pennec, G., Steffen, R., Ammar, M.S.A., Wiens, M., Müller, I.M. & Schröder, H.C. (2003b) Molecular mechanism of spicule formation in the demosponge Suberites domuncula: silicatein - collagen - myotrophin. Progress Molec. Subcell. Biol. 33,195-22.
Pancer, Z., Kruse, M., Schäcke, H., Scheffer, U., Steffen, R., Kovács, P. & Müller, W.E.G. (1996). Polymorphism in the immunoglobulin-like domains of the receptor tyrosine kinase from the sponge Geodia cydonium. Cell Adhesion and Commun. 4, 327-339.
Pronzato, R., Bavestrello, G., Cerrano, C., Magnino, G., Manconi, R., Pantelis, J., Sara, A. & Sidri, M. (1999) Sponge farming in the Mediterranean Sea: new perspectives. Mem. Queensl. Mus.44: 485-491.
Romeis, B. (1989) Mikroskopische Technik. Urban/Schwarzenberg, München.
Schütze, J., Krasko, A., Diehl-Seifert, B., Müller, W.E.G. (2001) Cloning and expression of the putative aggregation factor from the marine sponge Geodia cydonium. J. Cell Sci.114, 3189-3198.
Sunde RA (2001) Regulation of selenoprotein expression. In Selenium - its Molecular Biology and Role in Human Health(Hatfield, D.L., ed.), pp. 81-96. Kluwer Academic Publishers, Boston.
Wiens, M., Koziol, C., Hassanein, H.M.A., Batel, R. and Müller, W.E.G. (1998). Expression of the chaperones 14-3-3 and HSP70 induced by PCB 118 (2,3',4,4',5-pentachlorobiphenyl) in the marine sponge Geodia cydonium. Mar. Ecol. Prog. Ser. 165,247-257.

## Claims

1. Culture medium for the land-based aquaculture or *in vitro* culture of sponges, comprising between 10 and 1000 µM Se.

2. Culture medium according to claim 1 further comprising between 10 and 100 µM Si, and between 10 and 100 µM Fe3+.

3. Culture medium according to claim 1 or 2 wherein said medium is based on freshwater, brackish water, natural seawater or artificial seawater with a salinity ranging from 0 to 50 ‰.

4. Culture medium according to any of claims 1 to 3 wherein silicon is added to the medium as silicon salts such as sodium metasilicate, silicon containing natural materials such as silicon sand or artificial materials such as glass, or silicon-enriched commercial media.

5. Culture medium according to any of claims 1 to 4 wherein the ferric iron is added to the medium as either ferric iron salts such as ferric citrate, ferric iron containing natural or artificial materials, or ferric iron-enriched commercial media.

6. Culture medium according to any of claims 1 to 5 wherein the selenium is added to the medium as either selenium salts such as sodium selenite, selenium containing natural or artificial materials, or selenium-enriched commercial media such as selenite-broth.

7. Culture medium according to any of claims 1 to 6 wherein said medium further contains an organic food source, such as, for example the marine microalga *Phaeodactylum tricornotum,* or commercial cell culture media such as DMEM or RPI.

8. A method for biogenic silica formation comprising cultivating sponges, sponge fragments, sponge primmorphs, sponge cells in a culture medium according to any of claims 1 to 7, and/or comprising an enzymatic biosilica formation comprising sponge-derived enzymes comprising incubation in a culture medium according to any of claims 1 to 7.

9. The method according to claim 8, comprising an induction of the silicatein associated protein according to SEQ ID No. 1 and/or SEQ ID No. 3.

10. Silicatein associated protein comprising the amino acid sequence according to SEQ ID No. 1 and/or SEQ ID No. 3, and biologically functional variants thereof.

11. Nucleic acid molecule encoding for the silicatein associated protein according to claim 10, in particular according to the nucleic acid sequence according to SEQ ID No. 2 or SEQ ID No. 4.

12. A method for producing a culture medium for the land-based aquaculture or *in vitro* culture of sponges, comprising adding of between 10 and 1000 µM Se.

13. Use of a culture medium according to any of claims 1 to 7 for biogenic silica formation comprising cultivating sponges, sponge fragments, sponge primmorphs, sponge cells in, and/or comprising an enzymatic biosilica formation comprising sponge-derived enzymes.

14. Use according to claim 13, wherein selenium is present in said medium at between 10 and 1000 µM Se.


SEQUENCE LISTING

<110>  BioTecMarin GmbH

<120>  Selenium-enriched liquid media for the cultivation of siliceous
       sponges and for biogenic silica production

<130>  FB15557

<160>  4

<170>  PatentIn version 3.3

<210>  1
<211>  243
<212>  PRT
<213>  Suberites domuncula

<400>  1

Met Ser Leu Ala Ser Tyr Ser Ser Ile Gln Asp Leu Val Leu Leu Ala
1               5                   10                  15

Phe Asp Cys Arg Tyr Cys Glu Gly Ala Met Asn Thr Val Thr Val Met
            20                  25                  30

Leu Val Val Val Leu Cys Glu Leu Ala Thr Ser Asn Ser Val Ser Asn
        35                  40                  45

Ser Val Leu Pro Thr Ser Val Ser Asn Ser Thr Ala Thr Pro Ser His
    50                  55                  60

Thr Val Asn Val Thr Ala Thr Pro Ser Lys Ala Val Asn Val Thr Ala
65                  70                  75                  80

Thr Pro Ser Lys Thr Val Asn Val Thr Ala Thr Pro Ser Gln Thr Val
                85                  90                  95

Asn Val Thr Ala Thr Pro Ser Gln Thr Val Asn Val Thr Ala Thr Pro
            100                 105                 110

Ser Gln Thr Val Asn Val Thr Ala Thr Pro Ser Lys Thr Val Lys Val
        115                 120                 125

Thr Thr Thr Pro Ser Lys Thr Val Asn Val Thr Ala Thr Pro Ser Lys
    130                 135                 140

Thr Val Asn Val Thr Ala Thr Pro Ser Gln Thr Val Asn Val Thr Ala
145                 150                 155                 160

Thr Thr Ser Thr Pro Ser Ser Thr Thr Thr Val Thr Ser Asn His Val
                165                 170                 175

Pro Ser Lys Ser Pro Ser Ala Ser Pro Ser Pro Ser Pro Ser Pro Pro
            180                 185                 190

Ser Pro Gln Thr Gly Pro Asn Val Gly Leu Ala Val Gly Leu Thr Phe
        195                 200                 205

Leu Val Val Ala Ile Leu Ile Ile Val Leu Gly Ile Gly Gly Tyr Val
    210                 215                 220

Ile Tyr Lys Arg Phe Gly Lys Tyr Gln Thr Ser Tyr Glu Met Leu Pro
225                 230                 235                 240

Thr Glu Gly

<210>  2
<211>  879
<212>  DNA
<213>  Suberites domuncula

<400>  2
ggcatgagct tagcttcagg aaagtctatg tcgctagcta gctatagctc gatccaggac      60

ctagttttgt tggcgtttga ctgtagatat tgtgagggag ccatgaacac tgtcactgtg     120

atgttggtgg tggtcctctg tgaattggcc acatccaact ctgtcagtaa ctctgtactg     180

cccacttcag taagcaactc aacagctact ccaagccaca cggtcaatgt gacggccact     240

ccaagtaaag cggtcaatgt cacggccact ccaagtaaaa cggtcaatgt gacggccact     300

ccaagtcaaa cggtcaatgt gacggccact ccaagtcaaa cggtcaatgt cacggccacc     360

ccaagtcaaa cggtcaatgt cacggccact ccaagtaaaa cggtcaaggt gacgaccact     420

ccaagtaaaa cggtcaatgt gacggccact ccaagtaaaa cggtcaatgt gacggccact     480

ccaagtcaaa cggtcaatgt gacagccacc accagtacac ccagtagtac gacaactgtc     540

acgagtaacc atgttcccag taagtcacca agcgccagtc catctcctag tccttcccct     600

ccctccccac aaactggtcc taacgttggt cttgctgttg gtctgacctt ccttgtggta     660

gccattctca ttattgtcct tggtattgga ggatatgtca tttacaagag gtttggtaaa     720

taccagacat cctatgaaat gctacccact gaaggctaaa ctatagactt gttttgttgc     780

tgaaaagtaa rtttatttca ttgatcaact ttcagtttgt acttgaattg aataaacaga     840

tttagagatt gacaaattaa aaaaaaaaaa aaaaaaaaa                            879

<210>  3
<211>  122
<212>  PRT
<213>  Suberites domuncula

<400>  3

Met Lys Val Ala Val Leu Phe Leu Thr Leu Cys Val Ser Leu Ala Ile
1               5                   10                  15

Gly Val Gly Glu Ile Lys Lys Ala Arg Leu Glu Ser Cys Ser Gly Arg
            20                  25                  30

Leu Asn Ser Leu Pro Lys Val Lys Arg Phe Ile Asn Asp His Leu Pro
        35                  40                  45

Asp Tyr Pro Asp Leu Glu Ile Lys Tyr Ile Gly Gly Ala Asp Pro Val
    50                  55                  60

Met Val Leu Met Asp Ala Glu Tyr Gln Glu Val Thr Arg Val Ser Val
65                  70                  75                  80

Ile Lys Met Ser Glu Gln Glu Ile Ile Asp Leu Leu Ala Ser His Asn
                85                  90                  95

Ile Val Lys Arg Ser Pro Glu Glu Ala Glu Ala Glu Glu Asp Thr Glu
            100                 105                 110

Pro Trp Glu Gln Asp Glu Arg Glu Glu Leu
        115                 120

<210>  4
<211>  660
<212>  DNA
<213>  Suberites domuncula

<400>  4
ctggagctcg cgcgcctgca ggtcgacact agtggatcca aagaattcgg cacgaggagt      60

tcgagtacaa gtaaaattaa gtctctggag aacatgaagg tagctgtact ttttctgaca      120

ctctgtgtgt cccttgccat tggtgttggg gagataaaga aggccaggct ggagagttgc     180

agtggatgac gtttgaattc gcttccaaag gtgaagcgtt tcatcaacga ccaccttcca     240

gactatcctg atttagagat taaatatatc ggtggggctg atccagttat ggtgctaatg     300

gatgcagaat atcaggaagt aacgcgtgtt tcggtaatta aaatgtctga acaagagatc     360

atcgatcttc ttgcatctca caacattgtc aaacgttcac ccgaggaagc tgaagctgag     420

gaggacacag agccttggga acaagatgaa cgtgaagagc tatagactga caaactctat     480

ttttcttcca tgtacatgct atagatatgt tacatttgtg cgtagcttga aatgatgtgt     540

ttgctttgaa tggcaatagc caagagcaga tactgaggaa attatagcta tcacatttgt     600

ggctatcatt aagttgttgt ttttttgtca aagtgaagtc attttgcatg caaaaaaaaa      660